# EUROPEAN PATENT SPECIFICATION

(11) **EP 1 242 626 B1**
(45) Date of publication and mention of the grant of the patent: **10.06.2009**
(21) Application number: 00989349.6
(22) Date of filing: 21.12.2000
(51) Int. Cl.: C12Q 1/68, C07H 21/04, G01N 31/22, C12M 1/36

(54) **DETECTION OF NUCLEIC ACIDS**
DETEKTION VON NUKLEINSÄUREN
DETECTION D'ACIDES NUCLEIQUES

(30) Priority: 21.12.1999 US 172798 P
(43) Date of publication of application: 25.09.2002
(73) Proprietor: ORTHO-CLINICAL DIAGNOSTICS, INC., Rochester, NY 14626-5101 (US)
(72) Inventor: MERCOLINO, Thomas, J., Stockton, NJ 08559-1910 (US); SERINO, German, Buenos Aires (AR); CONNELLY, Mark, C., Doylestown, PA 18901 (US)
(74) Representative: Fisher, Adrian John
(86) International application number: PCT/US2000/034726
(87) International publication number: WO 2001/046474

(56) References cited:
- EP-A- 0 745 690
- EP-A- 0 909 823
- WO-A-98/04740
- US-A- 5 670 316
- US-A- 5 670 316
- US-A- 5 837 832
- US-A- 5 863 722
- US-A- 5 863 722
- US-A- 5 962 218
- US-A- 5 989 823
- US-A- 5 989 823
- FULTON R.J. ET AL: 'Advanced multiplexd analysis with the FlowMetrix(TM) system' CLINICAL CHEMISTRY vol. 43, no. 9, September 1997, UNITED STATES, pages 1749 - 1756, XP002142645

### SEQUENCE LISTING

(1)GENERAL INFORMATION:
   (i) NUMBER OF SEQUENCES: 6
(2)INFORMATION FOR SEQ ID NO:1:
   (i) SEQUENCE CHARACTERISTICS:
      (A)LENGTH: 90 nucleotides
      (B)TYPE: Nucleic Acid
      (C)STRANDEDNESS: Single
      (D)TOPOLOGY: Linear
   (ii) MOLECULE TYPE: Probe
   (iii) HYPOTHETICAL: No
   (iv) ANTI-SENSE: No
   (v) ORIGINAL SOURCE: Synthetically prepared
   (vi) IMMEDIATE SOURCE: Same
   (vii) PUBLICATION INFORMATION: None
   (viii) SEQUENCE DESCRIPTION: SEQ ID:1: 
5'-TTTCCAAGTA AGCAATAACG TCAGCTCTTT CTTGTGGCTT CTTCATACCAG
CGAAAGACAT CTTAGTACCT GGCATGAACT TCTTTGGGT-3'
(2)INFORMATION FOR SEQ ID NO:2:
   (i) SEQUENCE CHARACTERISTICS:
      (A)LENGTH: 87 nucleotides
      (B)TYPE: Nucleic Acid
      (C)STRANDEDNESS: Single
      (D)TOPOLOGY: Linear
   (ii) MOLECULE TYPE: Probe
   (iii) HYPOTHETICAL: No
   (iv) ANTI-SENSE: No
   (v) ORIGINAL SOURCE: Synthetically prepared
   (vi) IMMEDIATE SOURCE: Same
   (vii) PUBLICATION INFORMATION: None
   (viii) SEQUENCE DESCRIPTION: SEQ ID:2: 
5′-ACCCAAAGAA GTTCATGCCA GGTACTAAGA TGTCTTTCGC TGGTATGAAG
AAGCCACAAG AAAGAGCTGA CGTTATTGCT TTGGAAA-3′
(2)INFORMATION FOR SEQ ID NO:3:
   (i) SEQUENCE CHARACTERISTICS:
      (A)LENGTH: 166 nucleotides
      (B)TYPE: Nucleic Acid
      (C)STRANDEDNESS: Single
      (D)TOPOLOGY: Linear
   (ii) MOLECULE TYPE: Target
   (iii) HYPOTHETICAL: No
   (iv) ANTI-SENSE: No
   (v) ORIGINAL SOURCE: Synthetically prepared
   (vi) IMMEDIATE SOURCE: Same
   (vii) PUBLICATION INFORMATION: None
   (viii) SEQUENCE DESCRIPTION: SEQ ID:3: 
5'-CTGCAGGCGC CAGCGTGGAC CATCAAGTAG TAATGAACGC ACGGACGAGG
ACATCATAGA GATTACACCT TTATCCACAG TTCTCGGTCT AACGCAGCAG
TCAGTGTATC AGCACCAGCA TCCGTAGTGA GTCTTCAGTG TCTGCTCCAG
GATCGTGGCG CTGCAG-3'
(2)INFORMATION FOR SEQ ID NO:4:
   (i) SEQUENCE CHARACTERISTICS:
      (A)LENGTH: 26 nucleotides
      (B)TYPE: Nucleic Acid
      (C) STRANDEDNESS: Single
      (D)TOPOLOGY: Linear
   (ii) MOLECULE TYPE: Primer
   (iii) HYPOTHETICAL: No
   (iv) ANTI-SENSE: No
   (v) ORIGINAL SOURCE: Synthetically prepared
   (vi) IMMEDIATE SOURCE: Same
   (vii) PUBLICATION INFORMATION: None
   (viii) SEQUENCE DESCRIPTION: SEQ ID:4:
      5'-CGCCAGCGTG GACCATCAAG TAGTAA-3'
(2)INFORMATION FOR SEQ ID NO:5:
   (i) SEQUENCE CHARACTERISTICS:
      (A)LENGTH: 26 nucleotides
      (B) TYPE: Nucleic Acid
      (C)STRANDEDNESS: Single
      (D)TOPOLOGY: Linear
   (ii) MOLECULE TYPE: Primer
   (iii) HYPOTHETICAL: No
   (iv) ANTI-SENSE: No
   (v) ORIGINAL SOURCE: Synthetically prepared
   (vi) IMMEDIATE SOURCE: Same
   (vii) PUBLICATION INFORMATION: None
   (viii) SEQUENCE DESCRIPTION: SEQ ID:5:
      5'-CACGATCCTG GAGCAGACAC TGAAGA-3'
(2)INFORMATION FOR SEQ ID NO:6:
   (i) SEQUENCE CHARACTERISTICS:
      (A)LENGTH: 30 nucleotides
      (B) TYPE: Nucleic Acid
      (C)STRANDEDNESS: Single
      (D)TOPOLOGY: Linear
   (ii) MOLECULE TYPE: Probe
   (iii) HYPOTHETICAL: No
   (iv) ANTI-SENSE: No
   (v) ORIGINAL SOURCE: Synthetically prepared
   (vi) IMMEDIATE SOURCE: Same
   (vii) PUBLICATION INFORMATION: None
   (viii) SEQUENCE DESCRIPTION: SEQ ID:6:
      5'-CTGCGTTAGA CCGAGAACTG TGGATAAAGG-3'

## Description

### FIELD OF THE INVENTION

The present invention is directed to the detection of nucleic acids. More specifically, it is directed to detection of target nucleic acids using substrate-immobilized oligonucleotides and methods that provide a detectable signal associated with the substrate or substrate-immobilized oligonucleotide and a detectable signal correlated with the target nucleic acid hybridized thereto.

### BACKGROUND OF THE INVENTION

The detection of nucleic acids is widely employed for determining the presence and copy number of specific genes, known sequences, and identifying and quantifying viral, prokaryotic and eukaryotic pathogens in clinical and environmental samples. An important characteristic of nucleic acids is their ability to form sequence-specific hydrogen bonds with a nucleic acid having a complementary nucleotide sequence. This ability of nucleic acids to hybridize to complementary strands of nucleic acids has been used to advantage in what are known as hybridization assays, and in DNA purification techniques.

In a hybridization assay, a nucleic acid having a known sequence is used as a probe that hybridizes to a target nucleic acid having a complementary nucleic acid sequence. Labeling the probe allows detection of the hybrid and, correspondingly, the target nucleic acid.

Analytical assays rely on the generation of an analyte-dependent change in a detectable signal, ideally, in homogenous format. An analytical system that allows homogenous and simultaneous determination of multiple target nucleic acids (multiplexing) is highly desirable. Such systems are not available for nucleic acid analysis.

Methods of detecting target nucleic acids using DNA arrays on chips represent a significant advance in the art. Such methods are described in U.S. Patents 5,856,101; 5,837,832; 5,658,802 and 5,571,639.

Flow cytometers have been used as microparticle analyzers in multiplexed particle-based assays (Fulwyler, et al., Methods Cell Biology, Second Edition, Academic Press, Vol 33, 613 (1990), McHugh, Methods Cell Biology, Second Edition, Academic Press, Vol 42, 575 (1994), McHugh, et al. Cytometry 29, 106 (1997). In general, micron-size spherical particles (beads) are used as a solid phase serving as the origin of a fluorescence signal, the intensity of which is a function of the concentration of an analyte. The fluorescence signal is detected as the particles are analyzed individually by the flow cytometer as they pass single file through a multiparameter detector. When different assays are performed on particles that are distinguishable from each other by size or fluorescence intensity (or a combination of both), each fluorescence intensity or size represents a different assay. Fluorescence labeling of microspheres has allowed a multiplex capacity of up to 512 simultaneous different assays (Chandler et al., ISAC XIX International Congress; Colorado Springs, Colorado USA, 28 February - 5 March, 1998). The flow cytometric particle assays are potentially homogeneous because signals are recorded in conjunction with a triggering particle event. Signals not associated with a triggering particle event, which otherwise would interfere with the assay, are ignored by the instrument.

Methods for sizing DNA fragments using flow cytometry, are described in Castro et al., Anal Chem 65, 849 (1993), Goodwin et al., Nucleic Acids Res 21, 803 (1993), Petty et al., Anal Chem 67, 1755(1995) and U.S. Patent No. 5,558,998. A compound capable of fluorescence (fluorophore or fluor) and capable of binding stoichiometrically to DNA fragments generates a signal that is detected as the fragments flow individually through the detection apparatus of the flow cytometer. A laser beam excites the fluorophore at a specific wavelength and the emitted fluorescence is measured as a pulse of intensity proportional to the number of fluorophore molecules bound to the DNA fragment, and therefore, to the length of the fragment. However this method require that all signal events be detected and recorded, not just those associated with a triggering event. Further, this method provides no sequence information, or gene identity, only the relative size of the fragment. Also, it can not be done as a homogeneous method.

A method for detecting DNA sequences using flow cytometry based on competition between labeled probes and target for binding to an oligonucleotide bead-set is described in U.S. Patent 5,736,330 to Fulton (the '330 patent), also Spain, IVD *Technology Magazine* (1998), and McDade et al., Medical Device & Diagnostic Industry Magazine (1997).

A method for determining duplex DNA by flow cytometric fluorescence detection based on binding of duplex DNA to a bead-linked intercalator is described in U.S. 5,582,984 to Bieniarx et al. (the '984 patent).

The '330 patent describes a useful advance in the art; however, it requires the use of a discrete labeled probe for each DNA target, and detection of target using an indirect competitive binding method rather than a more advantageous direct binding method.

The methods described in the '984 patent are useful for detecting the presence of duplex DNA, but do not permit detection of specific target nucleic acids. Intercalating dye is not free in solution, but is linked to a bead; the signal intensity is limited, as multiple dye molecules are not free to bind to a single molecule of duplex DNA.

WO 98/04740 describes methods of detecting a nucleic acid. The methods involve contacting the nucleic acid with one or more types of particles having oligonucleotides attached thereto. A detectable change is brought about as a result of the hybridization of the oligonucleotides on the particles to the nucleic acid.

US 5,989,823 describes a method for homogeneous detection of a target through nucleic acid ligand-ligand beacon interaction. The methods allow the presence of a target in a test mixture or the concentration of such target to be determined by fluorescence emission measurement.

EP 0 745 690 describes unimolecular and bimolecular hybridization probes for the detection of nucleic acid target sequences. Hybridization of the target and probe is measured using a detectable label.

These and other prior art methods for determining a target nucleic acid have gained wide acceptance and utility. However, a need still remains for a method that is specific, quantitative, homogenous, rapid, capable of automation, allows determination and identification of multiple target nucleic acids. Ideally, such a method would also be sufficiently sensitive so that target amplification is either not required or requires only a small number of amplification cycles when the target is already present in multiple copies.

### SUMMARY OF THE INVENTION

These needs have been achieved with the present invention.

The invention relates to a method for determining the presence or amount of a target nucleic acid comprising:
(A) forming a mixture comprising
   i) the target nucleic acid,
   ii) target-specific particles, wherein a target-specific particle has immobilized thereto an oligonucleotide comprising a nucleic acid sequence complementary to at least a portion of the target nucleic acid, wherein the oligonucleotide comprises a second compound capable of fluorescence and the particle or the oligonucleotide comprises a fluorescence quenching compound in sufficient proximity to said second compound to quench the fluorescence of said second compound prior to hybridization of the oligonucleotide to the target nucleic acid, and wherein the particle
      (a) comprises a first compound or plurality of compounds capable of producing a distinct fluorescence signal corresponding to the particle, or
      (b) is capable of scattering electromagnetic radiation of wavelength greater than or equal to about 200nm and comprises a first compound or plurality of compounds capable of producing a distinct fluorescence signal corresponding to the particle;
(B) heating the mixture to denature duplex nucleic acid then cooling the mixture to allow hybridization of target nucleic acid to immobilized oligonucleotide;
(C) exposing the mixture to a detection device capable of detecting fluorescence and, optionally, scattered electromagnetic radiation;
(D) detecting the fluorescence of a first compound or a plurality of compounds of a particle, or detecting both the fluorescence of a first compound or a plurality of compounds of a particle and the scattered electromagnetic radiation of a particle; and
(E) detecting the fluorescence signal of the second compound as a measure of the presence or amount of the target nucleic acid.
and a method for determining the presence or amount of a plurality of distinct target nucleic acids comprising:
(A) forming a mixture comprising
   i) the target nucleic acids,
   ii) target-specific particles, wherein a target-specific particle has immobilized thereto an oligonucleotide comprising a nucleic acid sequence complementary to at least a portion of one distinct target nucleic acid, wherein the oligonucleotide comprises a second compound capable of fluorescence and the particle or the oligonucleotide comprises a fluorescence quenching compound in sufficient proximity to said second compound to quench the fluorescence of said second compound prior to hybridization of the oligonucleotide to the target nucleic acid, and wherein the particle
      (a) comprises a distinct first compound or plurality of compounds capable of producing a distinct fluorescence signal corresponding to the particle, or
      (b) is capable of scattering electromagnetic radiation of wavelength greater than or equal to about 200nm and comprises a distinct first compound or plurality of compounds capable of producing a distinct fluorescence signal corresponding to the distinct particle,
   iii) a compound capable of binding to duplex nucleic acid and producing a detectable optical signal when bound thereto;
(B) heating the mixture to denature duplex nucleic acid then cooling the mixture to allow hybridization of target nucleic acid to immobilized oligonucleotide;
(C) exposing the mixture to a detection device capable of detecting fluorescence and, optionally, scattered electromagnetic radiation;
(D) detecting the fluorescence of a first compound or a plurality of compounds of a particle, or detecting both the fluorescence of a first compound or a plurality of compounds of a particle and the scattered electromagnetic radiation of a particle; and
(E) detecting the fluorescence signal of the second compound as a measure of the presence or amount of each distinct target nucleic acid.

The mixture can be heated to denature duplex nucleic acid, then cooled to allow hybridization of the target nucleic acid to the immobilized oligonucleotide or, if desired, target nucleic acid can be denatured prior to combination with immobilized oligonucleotide.

The method involves a step for producing a detectable signal, preferably an optical signal, such as fluorescence, correlated with hybridized target and immobilized oligonucleotide probe. In one embodiment, a second compound capable of binding to duplex nucleic acid and producing a detectable fluorescence signal when bound thereto is combined with the mixture. In a different embodiment, the oligonucleotide may have linked thereto, but not necessarily, a second compound capable of fluorescence, and the mixture is contacted with a single-strand specific endonuclease. In another embodiment, the oligonucleotide or the substrate comprises a second compound capable of fluorescence. Also, the substrate or oligonucleotide comprises a fluorescence quenching compound in sufficient proximity to the second compound to quench fluorescence of the second compound prior to hybridization of target nucleic acid to the immobilized oligonucleotide. In another embodiment, the oligonucleotide attached to the bead comprises, or is capable of comprising, a sequence representing one complement of a known restriction endonuclease target site. Sample or amplified nucleic acid sequences are allowed to hybridize to the particle-linked oligonucleotide in the presence of a restriction endonuclease capable of cleaving the site. Optionally, the restriction endonuclease can be added later. If the appropriate target nucleic acid is present, the complete restriction site is formed as a result of heteroduplex formation, and the restriction enzyme cleaves the nucleic acid releasing all or part of the labeled double-strand sequence from the particle. This reduces or eliminates the nucleic acid specific fluorescence signal associated with that particular triggering event.

Using any of the above embodiments for producing a detectable signal, or signal change correlated to target hybridized to immobilized oligonucleotide probe, the mixture, in the case of particle substrates, is exposed to or introduced into a device or instrument capable of detecting the fluorescence of a first compound or a plurality of compounds of a particle, or detecting both the fluorescence of a first compound or a plurality of compounds of a particle and the scattered electromagnetic radiation of a particle, thereby distinguishing particles from background. The fluorescence signal from the second compound, the target-correlated fluorescence, is also detected as a measure of the presence or amount of the target nucleic acid.

Any particle analysis method or device capable of distinguishing particles from background and one target-specific particle from another by detecting particle-associated scattering and/or fluorescence, and which is capable of detecting target-correlated signal, can be used in the practice of this invention. Laser flow cytometric methods are preferred. A laser flow cytometer useful in the practice of the present invention, is the ORTHO CYTORONABSOLUTE® Flow Cytometer from Ortho-Clinical Diagnostics, Inc., Raritan, NJ. Fluorescence microscopic methods and devices can also be employed. Laser scanning methods and devices also can be used, for example, the Compucyte Laser Scanning Cytometer from Compucyte Corporation, Cambridge, MA. Methods that permit spatial resolution of substrate-immobilized oligonucleotides can be used; laser scanning methods are particularly suitable. Methods and devices that are capable of distinguishing particles over background and resolving scattering or fluorescence signal from target-specific particles, and detecting target-correlated signal, as described above, that do not rely on physical separation of individual particles also can be employed.

As discussed above, the mixture, optionally, can be heated to denature duplex nucleic acid then cooled to allow hybridization of the target nucleic acid to the immobilized oligonucleotide or, if desired, the target nucleic acid can be heated prior to combination with immobilized oligonucleotide.

The method for determining multiple target nucleic acids, involves a step for producing a detectable signal, preferably an optical signal, most preferably fluorescence, correlated with target hybridized to immobilized oligonucleotide. A second compound capable of binding to duplex nucleic acid and producing a detectable fluorescence signal when bound thereto is combined with the mixture, or the oligonucleotide has linked thereto a second compound capable of fluorescence and the mixture is contacted with single-strand specific endonuclease, or the oligonucleotide or substrate comprises a second compound capable of fluorescence and the substrate or oligonucleotide comprises a fluorescence quenching compound in sufficient proximity to the second compound to quench fluorescence of the second compound prior to hybridization of target nucleic acid to immobilized oligonucleotide.

Using the above embodiments for producing a detectable signal correlated to target hybridized to immobilized probe, the mixture, in the case of particulate substrates, is exposed to, or introduced into a device or instrument, as described above, capable of detecting the fluorescence of a first compound or a plurality of compounds of a particle, or detecting both the fluorescence of a first compound or a plurality of compounds of a particle and the scattered electromagnetic radiation of a particle and detecting the fluorescence signal from the second compound as a measure of the presence or amount of each distinct target nucleic acid. This can be accomplished because the target-specific particles can be distinguished by their specific light scattering and/or fluorescence. The second compound that provides target-correlated fluorescence signal, therefore, can be the same for each target. However, distinct second compounds for each target can be used if desired.

The resulting fluorescence signal is proportional to the amount of target hybridized to immobilized oligonucleotide. Fluorescence that may be associated with target nucleic acid and non-target nucleic acid free in solution, not hybridized to immobilized probe, does not contribute substantially, if at all, to the measured target-correlated signal.

A high degree of specificity and sensitivity can be obtained using long oligonucleotide probe sequences, which also permits use of stringent hybridization conditions.

In another aspect, the invention relates to a kit for detecting a target nucleic acid comprising in the same or separate containers:
(A) a particle
   (a) comprising a compound or plurality of compounds capable of producing a fluorescence signal corresponding to the particle, or
   (b) capable of scattering electromagnetic radiation of wavelength greater than or equal to about 200nm and comprising a compound or plurality of compounds capable of producing a fluorescence signal corresponding to the particle; and
(B) an oligonucleotide comprising
   (a) a nucleic acid sequence complementary to at least a portion of the target nucleic acid,
   (b) a first compound capable of fluorescence, and
   (c) a second compound capable of quenching the fluorescence of the first compound.

Also described herein is a method for detecting an amplified nucleic acid sequence of a biological sample comprising the steps of :
combining the biological sample containing said nucleic acid sequence with microparticles, a thermostable fluorophore, and a thermostable polymerase;
amplifying the selected segment of the nucleic acid by the polymerase chain reaction; and
detecting the nucleic acid sequence.
The thermostable fluorophore is selected from the group consisting of SYBR green I, ethidium bromide and propidium iodide, preferably the thermostable fluorophore is SYBR green I.

Further described herein is a method for detecting an amplified nucleic acid sequence of a biological sample comprising the steps of :
combining the biological sample containing said nucleic acid sequence with microparticles and a thermostable polymerase;
amplifying the selected segment of the nucleic acid by the polymerase chain reaction;
combining the amplified product of step (b) with a fluorophore; and
detecting the nucleic acid sequence.
The fluorophore is selected from the group consisting of concentrated picogreen, SYBR green I, ethidium bromide and propidium iodide, and is preferably concentrated picogreen.

Also described herein is a method for detecting an amplified nucleic acid sequence of a biological sample comprising the steps of :
amplifying the nucleic acid sequence in the presence of a thermostable polymerase by the polymerase chain reaction;
combining the amplified product of step (a) with a fluorophore and microparticles; and
detecting the nucleic acid sequence.
The fluorophore is selected from the group consisting of concentrated picogreen, SYBR green I, ethidium bromide and propidium iodide and is preferably concentrated picogreen.

Also described herein is a DNA chip comprising oligonucleotide arrays comprising target-specific loci and replicate loci. The chip or slide is useful for determining the presence or amount of one or more specific target sequences. The chip comprises between about 5 and about 20 target -specific loci and between about 5 to about 20 replicate loci, more preferably between about 100 to about 1000 target-specific loci and between about 10 to about 100 replicate loci for each target.

There is further described a method of detecting one or more target DNA sequences using a DNA chip comprising:
contacting a DNA chip, to which one or more oligonucleotides corresponding to the one or more target sequences to be detected have been anchored, with the sample containing said one or more target sequences, generally from about 30 pg to about 200 ng or higher of fluorescently labeled DNA; and
detecting the presence or amount of the one or more specific target sequences.

The fluorophore is selected from the group consisting of acridine orange, propidium iodide, ethidium bromide, mithramycin, chromomycin, olivomycin, see also, U.S. Patent Nos. 5,049,490 and 5,563,037. Preferred compounds include, Hoechst H33258, Hoechst H33342, DAPI (4',6-diamidino-2-phenylindole), and from Molecular Probes, TOPRO, TOTO, YOPRO, YOYO, SYBR GREEN I, and Picogreen, and others as commonly used in the art; the fluorophore is preferably concentrated Picogreen.

All of the above methods or kits, can be configured for determining a single target nucleic acid or a plurality of target nucleic acids.

### BRIEF DESCRIPTION OF THE FIGURES

FIG. 1 is a schematic diagram depicting the hybridization and detection of target nucleic acid hybridized to particle-immobilized oligonucleotide.
FIG. 2A is a plot of forward-angle vs right-angle light scattering (particle size selection) thiazole orange as target DNA indicator, in presence of CTDNA, target DNA was not present.
FIG. 2B is a plot of forward-angle vs right-angle light scattering (particle size selection), thiazole orange as target DNA indicator, 1000 femtomoles of target DNA in an excess of CTDNA.
FIG. 2C is a plot of forward-angle light scattering vs green fluorescence, thiazole orange as target DNA indicator in the presence of CTDNA, target DNA not present.
FIG. 2D is a plot of forward-angle light scattering vs green fluorescence, thiazole orange as target DNA indicator, 1000 femtomoles of target DNA in an excess of CTDNA.
FIG. 2E is a plot of the distribution of events vs green fluorescence, thiazole orange as target DNA indicator in the presence of CTDNA, target DNA not present.
FIG. 2F is a plot of the distribution of events vs green fluorescence, 1000 femtomoles of target DNA in an excess of CTDNA.
FIG. 3 is a plot of the mean green fluorescence vs copy number of double-strand target DNA.
FIG. 4 shows a schematic of nuclease protection and detection of particle-associated target nucleic acid.
FIG. 5 is a plot of mean channel orange fluorescence (stained with streptavidin-conjugated R-phycoerythrin, nuclease protected dsDNA captured on beads) vs copy number of double-strand target DNA.
FIG. 6 is a plot of mean channel fluorescence vs PCR amplification cycle number.

### DETAILED DESCRIPTION OF THE INVENTION

### General Discussion

For the purposes of the present invention, the term "substrate" represents any particulate or non-particulate material to which or upon which an oligonucleotide can be immobilized.

In a general sense, the methods of the present invention for detecting a target nucleic acid rely on a set of measurements of a first parameter or parameters in order to identify an assay. A corresponding set of measurements of a second parameter or parameters is made to determine the presence of, or to quantify a target nucleic acid. The assay identification and target determination parameters, are used to generate a correlated data list. These parameter measurements can be made sequentially. Each set of measurements is referred to as an event. Thus, a data list can appear as follows:

| Event | Parameter X | Parameter Y | parameter Z |
|---|---|---|---|
| 1 | X1 | Y1 | Z1 |
| 2 | X2 | Y2 | Z2 |
| 3 | X3 | Y3 | Z3 |
| ... | ... | ... | ... |
| n | Xn | Yn | Zn |

Such data lists are typically obtained using laser scanning cytometry or flow microfluorimetry. They are generally referred to as "list-mode data."

A range of values for a single parameter X ("A") can be used to define an assay for a first target nucleic acid. Another range of values for X ("B") can be used to define an assay for a second target nucleic acid. By this means, both assays can be carried out simultaneously. Whenever an event is correlated with a value for X, in range A, the measurement of parameter Z would be for the first target nucleic acid. Whenever an event is correlated with a value for X, in range B, the measurement of parameter Z would be for the second target nucleic acid. The number of assays that can be carried out simultaneously is limited only by the number of discrete ranges that can be detected within the parameter X being used to specify an assay for each target nucleic acid. A high degree of certainty can be obtained that events belong to a particular assay class by setting broad ranges for A, B, etc.

The number of assays that can be carried out simultaneously can be increased if more than one parameter is used to specify an assay. A range of values for parameter X ("A") in conjunction with a range of values for parameter Y ("Q") can be used to specify an assay for a first target nucleic acid. Another range of values for X ("B") in conjunction with another range of values for parameter Y ("R") can be used to specify an assay for a second target nucleic acid. By this means, both assays can be carried out simultaneously. Whenever an event is correlated with a value for X, in range A, and Y, in range Q, the measurement of parameter Z would be for the first target nucleic acid. Similarly, whenever an event is correlated with a value for X, in range B, and Y, in range R, the measurement of parameter Z would be for the second target nucleic acid. When using multiple parameters to identify an assay, the number of assays that can be carried out simultaneously is no longer limited by the number of discrete ranges of an individual parameter, since a specific combination of parameters can be used to specify an assay for each target nucleic acid. Additionally, a higher degree of certainty can be obtained that events belong to a particular assay class by setting broad ranges for A, B, Q, R, and so on.

In laser scanning cytometry or flow cytometry, the preferred parameters for classifying multiple simultaneous assays are forward light scatter, side scatter, or a fluorescence parameter(s) distinct from that used to detect signal from hybridized nucleic acids. For DNA chips, the preferred parameters for classifying multiple assays are spatial dimensions, x and y coordinates or positions on the surface of the chip.

A major advantage of the present invention, in addition to allowing multiple assays to be carried out simultaneously, is the ability to obtain replicate measurements of the same assay using the list mode data. For example, events 1 through n are classified as belonging to an assay for a first target nucleic acid, based upon measurements within a range of values for parameter X ("A") in conjunction with measurements within a range of values for parameter Y ("Q"). Events n+1 through p are classified as belonging to an assay for a second target nucleic acid, based upon measurements within a range of values for X ("B") in conjunction with measurements within a range of values for parameter Y ("R").

Statistical analysis can be performed to determine the mean and standard deviation of Z for the first assay by analyzing events 1 through n. Similarly statistical analysis can be performed to determine the mean and standard deviation of Z for the second assay by analyzing events n+1 through p. Thus, differences in signal between a target and control and between target levels can be expressed as differences between means of replicate measurements, thereby, enhancing the sensitivity of all assays performed. Events belonging to a particular assay class do not have to be measured sequentially. They have been shown as occurring sequentially for illustrative purposes only.

Another advantage is that a single parameter can be used to detect the signal from multiple target nucleic acids simultaneously. Thus, only one reporter element is necessary, regardless of the number of target nucleic acids to be determined. Conjugates of fluorescein isothiocyanate (FITC) are useful for signal generation. In preferred embodiments, an intercalating dye is used.

The determination of the presence or amount of double-strand or duplex nucleic acid in the presence of single-strand nucleic acid can be accomplished using a compound which upon binding or when bound to duplex nucleic acid, produces a detectable change in an optical property such as absorption or fluorescence (Ririe et al., Anal Biochem 245, 154 (1997), Wittwer et al., BioTechniques 22, 130 (1997), Yamamoto et al., European Patent Publication 0 643 140 A1 and U.S. Patent Nos. 5,049,490 and 5,563,037 to Sutherland et al.).

Nucleic acids can be determined using a "nuclease protection assay" as described in Sambrook et al. in Molecular Cloning: A Laboratory Manual, Vols. 1-3, 2nd ed., Cold Spring Harbor Laboratory Press, Cold Spring Harbor, NY (1989) and Thompson et al., Biol. Chem. 267, 5921 (1991). This method involves hybridization of a labeled, single-strand DNA probe to a target DNA or RNA molecule and subsequent hydrolysis of single-strand nucleic acid by a single-strand specific endonuclease, such as S1 nuclease. Hybridized duplex nucleic acid remains intact, protecting the labeled probe from hydrolysis by the endonuclease. The label can be a dye, a fluor, a radiolabeled molecule, an enzyme, and so on as recognized in the art, and appropriately detected. The assay can be quantitative for the target nucleic acid.

Nucleic acid amplification methods, such as the Polymerase Chain Reaction (PCR), often provide for the detection of a target nucleic acid using a labeled probe or, alternatively, a labeled primer that is extended into detectable product captured onto an immobilized complementary oligonucleotide probe. A wide variety of labels have been developed, Vlieger et al., Anal Biochem 205, 1 (1992), Yang et al., Blood 81, 1083 (1993). Fluoresceinated primers have been used in flow cytometric detection of bcl-2/MBR and IgH gene rearrangements, Barker et al., Blood 83, 1079 (1994).

Another method for determining a target nucleic acid involves linear amplification of signal from a target-specific oligonucleotide probe labeled with a fluor. The labeled probe, when hybridized to target, is hydrolyzed by a duplex nucleic acid-specific exonuclease, such as exonuclease III, which hydrolyzes duplex DNA (dsDNA) from the 3' terminus. Truncated hetero-duplex hybrids produced during hydrolysis are unstable. Shortened fragments of labeled probe dissociate. Fresh, intact, labeled probe can then hybridize to the target and a new round of hydrolysis occurs followed by dissociation of shortened, labeled probe and so on. The resulting probe fragments are then separated by electrophoresis and determined using a sequencing apparatus. (Okano et al. Anal Biochem 228, 101 (1995)).

Recently, Tyagi et al., Nature Biotechnolgy 14, 303 (1996), have described the use of, so-called, molecular beacons for the detection of nucleic acids. A molecular beacon is a target-specific oligonucleotide comprising a fluor, and in close proximity to the fluor, a quencher. Upon binding of target nucleic acid, the fluor and quencher become separated, and the resulting fluorescence is detected.

In the case of a plurality of distinct target nucleic acids, a target-specific substrate/particle is rendered specific to a distinct target nucleic acid by having immobilized thereto an oligonucleotide complementary to at least a portion of only one distinct target nucleic acid.

Target DNA can be in single-strand (ssDNA) or duplex form. If in duplex form, it can be treated, usually by heating, to denature the dsDNA prior to, during, or subsequent to combination with a substrate-immobilized oligonucleotide. The ssDNA target is then allowed to hybridize to substrate-immobilized oligonucleotide.

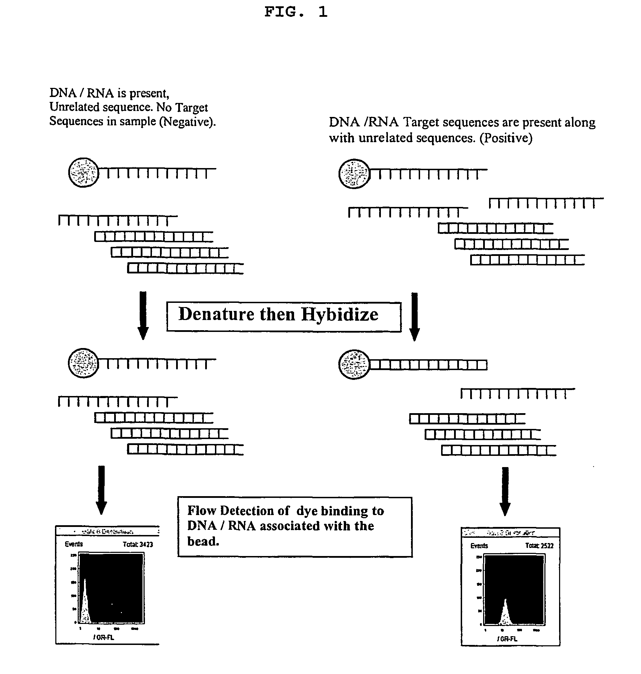

Any optical signal and method of producing it that is derivable from or associated with target nucleic acid hybridized to substrate-immobilized oligonucleotide can be used in the practice of this invention. As noted, in one embodiment, target-correlated signal is obtained by utilizing a fluor that binds to dsDNA and produces a detectable fluorescence signal when so bound. This is illustrated schematically in Figure 1. Alternatively, a fluorescence signal associated with target hybridized to substrate-immobilized oligonucleotide can be produced by utilizing a fluorophore linked to the oligonucleotide. When the substrate-immobilized oligonucleotide probe is hybridized to target nucleic acid to form duplex nucleic acid, single-strand specific endonuclease is not able to hydrolyze or substantially hydrolyze the oligonucleotide, or therefore, the portion of oligonucleotide having the fluor linked thereto. Thus, fluor linked to oligonucleotide remains associated with the substrate; target-correlated signal originating from the fluor that is associated with hybridized target is detected. Signal originating from fluor that is released by single-strand endonuclease mediated hydrolysis does not substantially contribute to the measured fluorescence. In a related embodiment, restriction endonuclease mediated hydrolysis can be used to release a fluorophore bound to the oligonucleotide, upon creation of a double strand DNA sequence that contains a restriction site when target hybridizes to substrate-immobilized oligonucleotide. In another embodiment, a fluorophore is bound to the oligonucleotide or the substrate so as to be sufficiently close to a compound capable of substantially quenching (by greater than or equal to about 50%) its fluorescence. If the fluor is linked to the substrate then the quencher is linked to the oligonucleotide. If the quencher is linked to the substrate then the fluor is linked to the oligonucleotide. Or fluor and quencher can both be linked to the oligonucleotide.
The ability of a fluor to produce a detectable signal only when bound to dsDNA, although preferred, is not a requirement of a fluor in order for it to be useful in practicing the invention.

### Particles and Particle-immobilized Oligonucleotide

Particle substrates of the present invention may be composed of any material or combination of materials that will enable covalent or non-covalent linking of oligonucleotide and/or other compounds such as fluorophores. They can be of a construction that allows compounds such as fluorophores to be encapsulated, as long as the fluorescence is detectable. They can be of a size and composition so as to scatter electromagnetic radiation. Preferably, the particles are composed of one or more polymers comprising ligands or functional groups that enable non-covalent or covalent bonding of oligonucleotides and/or other compounds directly or through suitable binding partners or linker groups. Preferably, they are substantially spherical and have a diameter in a range from about 0.3 microns to about 50 microns, more preferably 0.9 microns to about 15 microns and/or are capable of scattering electromagnetic radiation greater than or equal to about 200 nanometers. Polymeric particles for use in the practice of the invention can be prepared by methods known to the skilled artisan. See, for example, U.S. Patents 4,997,772, 5,149,737, 5,210,289 and 5,278,267 and references cited therein. Alternatively, suitable particles can be obtained for instance, from Bangs Labs, Fishers, IN and Spherotech, Libertyville, IL and others known to the skilled artisan.

The attachment of oligonucleotides to particulate and non-particulate substrates can be carried out using methods that are well known, as described, for example, in U.S. Patents 5,177,023, 4,713,326, 5,147,777, 5149,737, EP-B-0 070 687, WO-A-88/01302, and references cited therein. An oligonucleotide can have linked to it any desired compound or compounds as long as the compound or compounds do not substantially interfere with hybridization of the target nucleic acid. For example, a ligand such as biotin, a fluor, a fluorescence quenching compound, or other compound(s) can be so linked. An oligonucleotide can be linked to a particle at its 3' or 5' end.

An oligonucleotide probe can comprise any desired number of bases. In general, it can comprise a base length between about 5 to about 10⁵ nucleotide bases. Preferably, it comprises a base length between about 15 to about 40,000 bases, more preferably between about 30 to about 10,000 bases, and even more preferably between about 30 to about 1000 bases, and most preferably between about 30 to about 500 bases.

### Detection

An important aspect of the invention with particulate substrates is the use of methods and instrumentation capable of distinguishing particles and target-correlated signal over background, and target-specific particles, one from another, based on the light scattering and/or fluorescence properties of the particles. Target-specific particles are distinguishable, one from another, by the distinct differences in their light scattering properties and/or the distinct differences in the fluorescence signals derived from a fluorophore or plurality of fluorophores associated with each target-specific particle, which fluorescence signals are distinct from target-correlated fluorescence.

Light scattering by a particle depends on its size and/or refractive index, both of which can be modified as desired using well known methods. It is not necessary for a particle to be capable of scattering light. Particle discrimination can be achieved by incorporating, encapsulating, non-covalently or covalently bonding to a particle one or more compounds that are capable of producing distinct fluorescence.

Laser scanning cytometry has been used for distinguishing particles, such as blood cells. When a cell or group of cells (agglutinate) is scanned by the laser light beam, the illuminating light is scattered by the cell or group of cells; the intensity of scatter being a function of cell (or agglutinate) size and shape. For example, individual red blood cells scatter less light than small agglutinates, which in turn scatter less light than large agglutinates.

Similarly, when a cell or group of cells (agglutinate) is scanned by the laser light beam, the illuminating light can induce fluorescence from a fluor(s) associated with a cell or cells. If a fluor is relatively uniformly associated with a cell, the fluorescence intensity is related to agglutinate size. For example, individual red blood cells would fluoresce less than small agglutinates which in turn, would fluoresce less than large agglutinates.

Using scattered light and fluorescence in combination is more reliable than using either alone for discriminating different classes of agglutinates.

In flow cytometry, particles, such as blood cells, are introduced into the center of a fast moving fluid stream and forced to flow single file out a small diameter orifice at uniform speeds. The particles are hydrodynamically focused to the center of the stream by a surrounding layer of sheath fluid. The particles within the stream pass a measurement station where they are illuminated by a light source and measurements, in the case of red blood cells, are made at rates of 2.5 X 10² to 10⁶ cells per minute. Laser light sources are used in the measurement of particles; typical laser light sources used include argon ion lasers (UV, blue and green light), krypton lasers (yellow and red light), helium-cadmium lasers (UV and blue light), and helium-neon lasers (red light) .

In fluorescence microscopy, particles can be detected on a microscope slide or equivalent. Typically, they are illuminated by a white light source or a substantially monochromatic light source. Here too, laser light sources may be used as the source of the monochromatic light. The presence of particles may be assessed with the white light, and the associated fluorescence assessed with monochromatic light and appropriate filters. Visual or automated means may be used for one or both of these readings.

A preferred flow cytometer is capable of selecting for the detection of target-correlated signal associated with particles having a defined range of forward-angle and right-angle scattering signal intensity or particular fluorescence (Yang, et al. Blood 81, 1083 (1993), Barker et al. Blood 83, 1079-1085 (1994), Chandler, et al., ISAC XIX International Congress, Colorado Springs, Colorado USA, Fulton, et al., Clinical Chem 43, 1749 (1997), Fulwyler, et al., Methods Cell Biology, Second Edition, Academic Press, v 33, 613(1990), and McHugh, Methods Cell Biology, Second Edition, Academic Press, v 42, 575, (1994)). Data acquisition is initiated by light scattering and/or fluorescence associated with a particle. Selecting for signal associated with a particle enables the detection of target-correlated signal without interference from fluorescence originating from the bulk solution phase in which the particles are immersed. Thus, the signal/noise ratio is large. Target-correlated signal is proportional to the amount of target, and determination of multiple target nucleic acids is also possible using the preferred flow cytometric methods. Multiplex analysis of nucleic acids that are free in solution using flow cytometry has been described by Chandler et al., ISAC XIX International Congress; Colorado Springs, Colorado (1998), Fulton et al., Clin Chem 43 1749(1997), Fulwyler et al., Methods Cell Biology, 2nd Ed. 613(1990), and McHugh, Methods Cell Biology, 2nd Ed. 575(1990).

### DNA Binding Fluorophores

Numerous compounds capable of binding to dsDNA and producing a detectable signal when bound thereto or which can be chemically modified to produce a detectable signal are known and available to the skilled artisan. Included among them are dyes, antibiotics, and chemotherapeutic agents. They can be intercalating or non-intercalating; but, they must not bind substantially to the substrate to which oligonucleotide is immobilized. Specific examples include, but are not limited to, acridine orange, propidium iodide, ethidium bromide, mithramycin, chromomycin, olivomycin, see also, U.S. Patent Nos. 5,049,490 and 5,563,037. Preferred compounds include, Hoechst H33258, Hoechst H33342, DAPI (4',6-diamidino-2-phenylindole), and from Molecular Probes, TOPRO, TOTO, YOPRO, YOYO, SYBR GREEN I, Picogreen dsDNA Quantitation Reagent, and Thiazole Orange. Some compounds, such as YOYO, are virtually non-fluorescent until they bind dsDNA. Acridine orange has metachromatic properties that allow distinction between binding to ssDNA or dsDNA. For the purposes of the present invention, a compound either must not substantially bind to single strand immobilized oligonucleotide, but if it does, any resulting signal must be capable of being differentiated from that produced when the compound is bound to dsDNA.

### Single-Strand Specific Endonucleases, Restriction Endonucleases

Single-strand specific endonucleases that can be used in the practice of this invention include, but are not limited to, S1 endonuclease and mung bean endonuclease. Restriction endonucleases that can be used include, but are not limited to: *Acc65 I, Acc I, Aci I, Alu I, Apa I, ApaL, Ava I, Ava II, Bae I, BamH I, Bcg I, Bcl I, Bfa I, Bgl I, Bgl II, Bsa I, BsaJ I, Bsl I, BspH I, BsrG I, Bs t4C I, BssS I, BstE II, BstU I, BstX I, BstY I, Cla I, Dde I, Dpn I, Dpn II, Dra I, Dra III, EcoO109 I, EcoR I, EcoR V, Fau I, Fok I, Hae II, Hae III, Hha I, Hinc II, Hind III, Hinf I, Hpa I, Hpa II, Kpn I, Mbo I, Mlu I, Mnl I, Mse I, Msp I, Nae I, Nco I, Nde I, Nhe I, Nla III, Nru I, Pst I, Pvu I, Pvu II, Rsa I, Sac I, Sac II, Sal I, Sau3A I, Sca I, Sfi I, Sma I, SnaB I, Spe I, Sph I, Ssp I, Stu I, Sty I, Taq I, Xba I, Xcm I, Xho I, Xma I, and Xmn I*.

### Fluor and Quencher

In an embodiment wherein a fluorophore and a quencher are provided together, a fluorophore can be linked at or near a terminus of an oligonucleotide and a quencher can be linked at or near the other terminus, as in a stem-loop structure (molecular beacon) described by Tyagi et al., Nature Biotechnolgy 14, 303 (1996), Tyagi et al., Nature Biotechnolgy 16, 49 (1998), Giesendorf et al., Clin Chem 44, 482 (1998), and Estrada et al., Mol Cell Probes 12, 219 (1998). In a stem-loop structure the oligonucleotide comprises complementary nucleotide base pairs at both termini. Hybridization of the complementary base pairs results in formation of a closed loop with the fluorophore and quencher in sufficient proximity so that fluorescence is quenched. Upon hybridization of target nucleic acid to the oligonucleotide, the loop is opened and fluorophore and quencher become sufficiently separated to allow fluorescence.

In all cases, separation of substrate-immobilized oligonucleotide from the solution in which it is immersed is not required prior to detection. However, such separation may be carried out, if desired, using well-known methods such as filtration, centrifugation or magnetic separation.

In another aspect of the invention, target nucleic acid molecules can be hybridized under stringent conditions to microsphere-bound, oligonucleotide probes of complemetary sequence and hybridization detected by staining with a dsDNA-specific fluorescent dye. Target concentration and incubation time sufficient to saturate the microspheres can be used and signal measured at time points before and after saturation binding is achieved. No signal above background is observed for irrelevant target sequence. Maximum fluorescence signal is observed associated with the microsphere when the target sequence is complementary to the microsphere-bound oligonucleotide at every nucleotide position, and complete complementarity also yields the most rapid rise in signal as a function of analyte concentration. When a single base is mismatched in the target sequence, fluorescence signal is less than for fully complementary target sequence. Correspondingly, the rise in fluorescence signal with time is less rapid than for the fully complementary target sequence. In addition, maximum fluorescence and rate of rise in signal is a function of which nucleotide of the 3 possibilities was mismatched. Thus, this method provides an elegant approach to SNP detection, through measuring maximum fluorescence at saturation binding, or through measurement of signal increase with analyte concentration. Presumably, the position of a mismatch could also be mapped through this method, accordingly it should be possible to take advantage of this method in DNA sequencing applications.

While this disclosure describes detection of PCR products by mixing these with microspheres and intercalating dyes, further disclosed is the addition of the microspheres-immobilized oligonucleotide probes and the intercalating dyes to the PCR reaction before, as an alternative to after, thermocycling. In this embodiment of the invention, either the dye (in this embodiment, preferably a thermostable dye) or the microspheres, alone, could be in the mix prior to thermocycling, or alternatively, both may be present. Accordingly, sample handling can be even further reduced when measuring PCR products either in the homogeneous formats described herein above and in Example 6 hereof, or in other formats that might be used or developed by one skilled in the art.

### Materials and Methods for Examples

Unless indicated otherwise, the particles used in the examples were copolymers of (poly[styrene-co(p-vinylbenzylthio)proprionic acid] 97.6:2.4 molar ratio) prepared as described in U.S. Patent No. 5,149,737; 5,210,289 and 5,278,267. The particles were substantially spherical and approximately 1.7 micrometers in diameter. Coupling of oligonucleotide to the particles was carried out essentially as described in U.S. Patent No. 5,147, 777. Unless indicated otherwise, oligonucleotides were synthesized using procedures well known in the art. Two oligonucleotides used in the examples are identified below:

SEQ ID NO:1:

5′-TTTCCAAGTA AGCAATAACG TCAGCTCTTT CTTGTGGCTT CTTCATACCA
GCGAAAGACA TCTTAGTACC TGGCATGAAC TTCTTTGGGT-3′.

The above oligonucleotide was modified by linking biotin to the 5' terminus through two tetraethylene glycol (TEG-TEG) spacers with and without an aminodiol (ADL) linker at the 3' terminus for attachment to a particle as represented by the following: 
Oligo-1A:
Biotin-TEG-TEG-5'-TTTCCAAGTA AGCAATAACG TCAGCTCTTT CTTGTGGCTT
CTTCATACCAG CGAAAGACAT CTTAGTACCT GGCATGAACT TCTTTGGGT-3'-TEG-
TEG-ADL-Particle.
 and

Oligo-1B:
Biotin-TEG-TEG-5′-TTTCCAAGTA AGCAATAACG TCAGCTCTTT CTTGTGGCTT
CTTCATACCAG CGAAAGACAT CTTAGTACCT GGCATGAACT TCTTTGGGT-3′.

SEQ ID NO:2:

5′-ACCCAAAGAA GTTCATGCCA GGTACTAAGA TGTCTTTCGC TGGTATGAAG
AAGCCACAAG AAAGAGCTGA CGTTATTGCT TTGGAAA-3′.

### Hybridization Conditions

Hybridization of biotinylated oligonucleotide probe, (SEQ ID NO:1) to target DNA (SEQ ID NO:2) was carried out by incubating 100 fmoles of probe in 10 µL of 0.15M potassium chloride, 0.01M tris(hydroxymethyl)aminomethane (Tris), 1mM ethylenediaminetetraacetic acid (EDTA), pH 8.3 in the presence of 1 µg of calf thymus DNA. DNA was denatured by heating for 3 minutes at 96°C, and the mixture was then incubated at 65°C for 10. minutes to allow hybridization of target and oligonucleotide probe. Binding of the biotinylated oligonucleotide to streptavidin-coated beads (Bangs Laboratories) was carried out by supplementing the reaction mixture with 5x10⁴ beads in a final volume of 15 µL and incubated the mixture for 10 minutes at room temperature.

### Binding of Fluorophore

To a 500 µL aliquot of a working stock solution of fluorophore was added 10 µL of hybridized target-probe bead suspension and the mixture was incubated at room temperature for a minimum of 5 minutes. The working stock solution of fluorophore was prepared from the original preparation supplied by the manufacturer as follows: picogreen was diluted 1:10,000 with TE buffer, SYBR GREEN was diluted 1:200 with TE buffer, thiazole orange (Aldrich Chemical Company, Milwaukee, WI) 1 µg/mL in TE buffer, TOPRO-1, TOTO-1, YOPRO-1 and YOYO-1, all 0.5 µM in TE buffer.

### Nuclease Protection

After hybridization of bead-immobilized oligonucleotide probes (SEQ ID NO:1) to target DNA (SEQ ID NO:2), an 8 µL aliquot of the suspension was combined with 8 µL of 2x S1 nuclease buffer or 2x mung bean nuclease buffer (Promega, Madison, WI), digested with 1 unit of S1 nuclease or mung bean nuclease, and incubated at 30°C for 30 minutes. To the reaction mixture was added, a 1:10 dilution of streptavidin-conjugated phycoerythrin fluorophore in TE buffer to a final volume of 24 µL.

### Flow Cytometry

Flow cytometric analysis was performed using an Ortho CYTORONABSOLUTE® Flow Cytometer with Immunocount 2.2 software. Parameters for particle analysis were determined for each lot of bead-immobilized oligonucleotide. Gains and amplifiers for forward-angle scattering and right-angle scattering were setup such that beads of each size could be detected and resolved by the instrument. Fluorescence gain and amplifier settings in the CYTORONABSOLUTE® Flow Cytometer were adjusted to optimize hybridization-mediated fluorescence detection. Cluster analysis was used to determine the mean-peak channel fluorescence, since it allows thresholds of both fluorescence and another parameter (e.g., forward scattering or right scattering) to be preset, therefore permitting uniform criteria to be applied to different samples being analyzed. This procedure eliminates background noise resulting from particles presenting highly scattered fluorescence channel values.

The examples presented below utilize DNA as target nucleic acid. This is for illustrative purposes only. Wherever necessary, the methods of the present invention can be adapted readily for RNA targets, as would be known to the skilled practitioner.

### Example 1

### Detection Of Target DNA Hybridized To Particle-Immobilized Oligonucleotide: DNA Binding Fluorophores

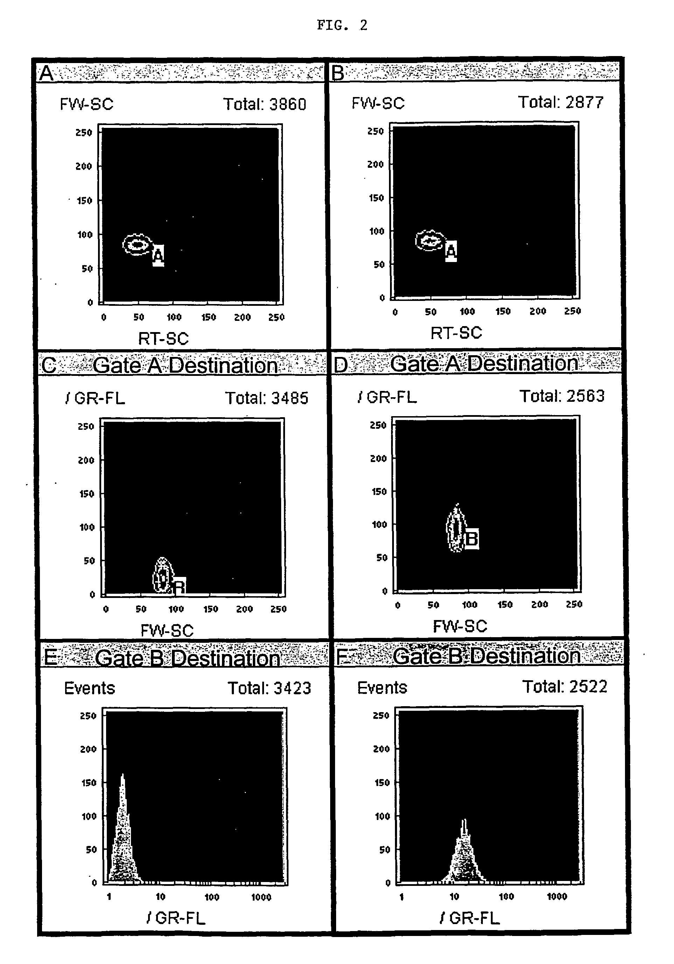

Fluorophores that bind to dsDNA were used to detect hybridized target DNA. The target DNA in this example (SEQ ID NO:2) was hybridized to bead-immobilized probe (SEQ ID NO:1) in the presence of excess calf thymus DNA. Fluorophore was combined with hybridized target-bead suspension and analyzed by flow cytometry. Results are illustrated in Figs. 2A-F using thiazole orange as the dsDNA binding fluorophore. The forward-angle scattering (FW-SC) x right-angle scattering (RT-SC) pattern of beads incubated with 1 µg calf thymus DNA in the absence (Fig. 2A) and presence (Fig. 2B) of target DNA is shown. Light-scatter gating of the beads by means of an image analysis software algorithm allows the analysis of select particles within a narrow range of FW-SC x RT-SC values, thus, eliminating particles outside the size range. The gated group of particles has the FW-SC x Green Fluorescence (GR-FL) pattern illustrated in Fig. 2, C and D, with thiazole orange as the dsDNA binding fluorophore, where further gating selects for the events used to calculate a mean channel fluorescence value. The group of particles selected in Fig. 2, C and D is represented in a fluorescence histogram in Fig. 2, E and F, where it can be seen that the distribution averages of a negative control (no target DNA) and positive (1000 fmoles of target) are clearly separated. Table 1 below shows the mean-peak channel fluorescence (MCF) for seven different fluorophores in the presence of CTDNA with and without added target DNA. Hybridizations were performed in a mixture containing 4x10⁴ beads comprising oligonucleotide probe (SEQ ID NO:1) immobilized thereto, with or without target (SEQ ID NO:2, 1000 fmoles) in presence of 1 microgram of CTDNA and 0.15M potassium chloride, 0.01M Tris, and 1mM (EDTA), pH 8.3, in a final volume of 10 microliters. The DNA was denatured at 96°C for 3 minutes and hybridized at 65°C.

**Table 1**

| | **MCF** | **MCF** |
|---|---|---|
| **Fluorophore** | **no target** | **1000 fmoles target** |
| Thiazole Orange | 22.5 | 87.4 |
| Picogreen | 27.1 | 103.7 |
| Sybrgreen | 15.8 | 51.5 |
| TO-PRO-1 | 19.3 | 101.6 |
| TOTO-1 | 13 | 49.1 |
| YO-PRO-1 | 20.6 | 59.8 |
| YOYO-1 | 36 | 63.6 |

There is no need to separate the particles associated with hybridized target and oligonucleotide from DNA free in solution as light-scatter gating of the particles by image analysis software allows analysis of a select group of those particles that present a cohesive, narrow range of forward angle scattering x right angle scattering values; limiting the analysis to only those particles of interest.

### Example 2

### Nuclease Protection

Hybridization of biotinylated particle-immobilized oligonucleotide probes (Oligo-1A) to target oligonucleotide (SEQ ID:2) protected the probe from hydrolysis by single strand specific DNA endonuclease. As in Example 1, particle-immobilized oligonucleotide and CTDNA were incubated together in the presence and absence of 1000 femtomoles of target DNA, followed by incubation with S1 nuclease. Biotin was released upon endonuclease hydrolysis of the oligonucleotide probe unless it was protected from hydrolysis by hybridization with target DNA.

An 8 microliter aliquot of streptavidin-linked fluor (streptavidin-phycoerythrin from Molecular Probes), diluted 1:10 in TE buffer, was added to 16 microliters of nuclease-treated sample, and incubated for 10 minutes at room temperature. Binding of streptavidin-linked fluor served as reporter for intact bead-linked oligonucleotide. The mixture was analyzed using flow cytometry. The mean channel fluorescence of phycoerythrin without target was 21.5, with 1000 femtomoles of target it was 34.7.

### Example 3

### Quantification of Target DNA: DNA Binding Fluorophore

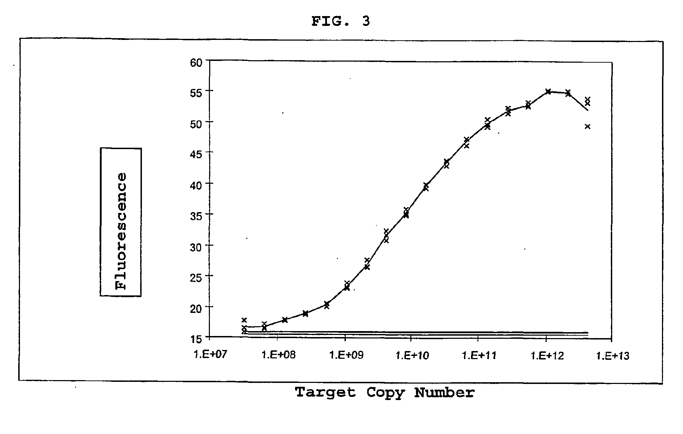

The fluorescence of the fluorophore, sybr green, bound to the hybrid of target DNA (SEQ ID NO:2) and bead-immobilized oligonucleotide (SEQ ID NO:1) is shown as a function of the copy number of the target in Fig. 3.

The fluorescence signal associated with bead-immobilized oligonucleotide is monotonically dependent on the concentration, demonstrating the ability to quantitatively determine the amount of target DNA over a concentration range of about 6 orders of magnitude. As little as 25.7 picograms of target dsDNA corresponding to 440 attomoles, or about 2.5 x 10⁸ copies of the 90-mer target, was clearly detected in a background of 0.8 µg non-specific calf thymus DNA. Thus the target DNA was readily detectable in the presence of about a 3.11 x 10⁴-fold excess of non-specific DNA. The results also show that the method is very sensitive over the concentration range; a two-fold increase in target DNA concentration was readily detectable within a concentration range between about 1.25 x 10⁸ to 1.09 x 10¹² copies of the target in a volume of 8 microliters. The average fluorescence obtained with thiazole orange, TOPRO-1, TOTO-1, YOPRO-1, YOYO-1, and picogreen, in each case, was also proportional to the concentration of the target DNA (data not shown).

### Example 4

### Quantification of Target DNA: dsDNA Binding Fluorophore,

In an alternative embodiment, a soluble, biotinylated oligonucleotide probe was allowed to hybridize to its target DNA in solution. The target DNA-biotin-oligonucleotide probe hybrid was then allowed to bind to streptavidin-beads. To the suspension was then added 500 microliters of a 1:200 dilution of picogreen and incubated 2 min at room temperature. The suspension was introduced into the flow cytometer. The calculated particle-associated mean channel fluorescence was proportional to the concentration of target DNA in the sample (data not shown).

### Example 5

### Quantification of Target DNA: Nuclease Protection

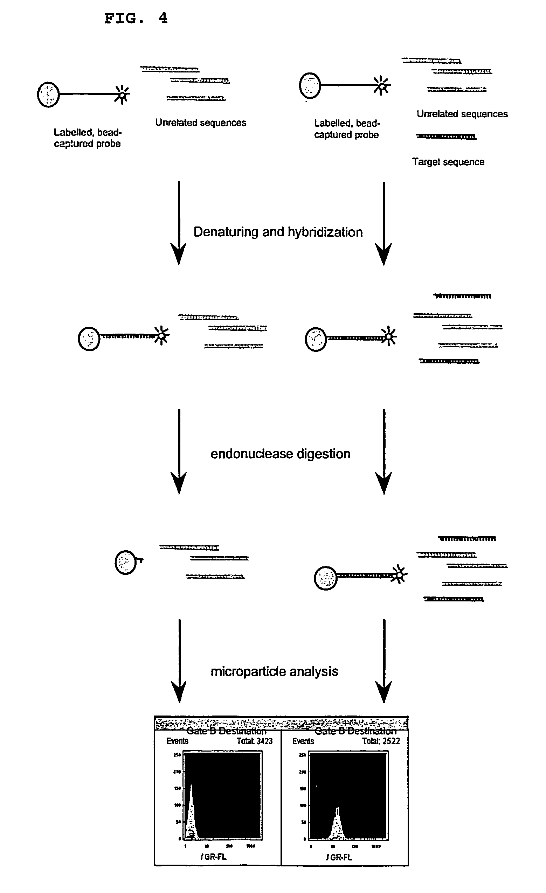

Figure 4 shows a schematic of a nuclease protection-based assay for detection of particle-associated target nucleic acid.

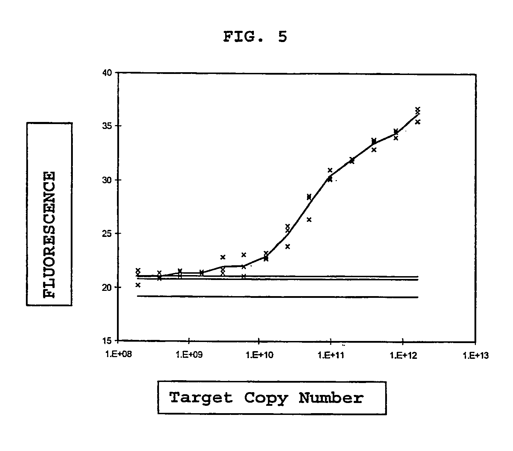

Protection of oligonucleotide probe from nuclease S1 digestion was proportional to the amount of target DNA. Fig. 5 shows the average fluorescence signal as a function of increasing single-stranded target DNA concentration. As little as 40.96 femtomoles (about 2.5 x 10¹⁰ copies) of the 90 base-pair DNA target (SEQ ID NO:2) was detected in about a 100-fold excess of non-target CTDNA.

### Example 6

### Detection of PCR Amplification Products: DNA Binding Fluorophore

To quantitatively measure low-abundance nucleic acids, PCR amplification of target DNA is frequently monitored as a function of amplification cycle number; the number of amplification cycles required to detect product being proportional to the target copy number.

In this example, 10 copies of target DNA were amplified from a plasmid-cloned DNA insert (SEQ ID NO. 3) using PCR. Target DNA (10 copies, SEQ ID:NO:3) was amplified in a volume of 100 microliters of an admixture containing CT (calf thymus) DNA, 5 micromolar NaOH, 4 milimolar MgCl₂, 18 mM Tris buffer, 54 mM KCl, 0.4 mM each primer (SEQ ID NO: 4 and SEQ ID NO: 5), 0.3 mM each dNTP, 0.108 microgram/microliter gelatin, 0.725 mM EDTA, 40 micromolar DTT, 9.5% glycerol, 0.02% Tween 20, 0.02% Nonidet P40.

### Target DNA

The target DNA having the following sequence consisted of a DNA fragment cloned in pUC18:

SEQ ID:NO:3:

5'-CTGCAGGCGC CAGCGTGGAC CATCAAGTAG TAATGAACGC ACGGACGAGG
ACATCATAGA GATTACACCT TTATCCACAG TTCTCGGTCT AACGCAGCAG
TCAGTGTATC AGCACCAGCA TCCGTAGTGA GTCTTCAGTG TCTGCTCCAG
GATCGTGGCG CTGCAG-3'

The underlined sequences correspond to the region amplified from the target DNA using the PCR primers described bellow (SEQ ID:NO:4 AND SEQ ID:NO:5) .

### PCR Primers

Primers used for target DNA amplification were synthetically prepared oligonucleotides according to the sequences:
SEQ ID NO:4: (forward primer)
   5'-CGCCAGCGTG GACCATCAAG TAGTAA-3'
SEQ ID NO:5: (reverse primer)
   5-'CACGATCCTG GAGCAGACAC TGAAGA-3'

PCR was carried out using standard thermal cycling protocols (cycles 1-5: 30s at 96°C: 60s at 68°C; cycles 6-40: 15s at 96°C; 60s at 68°C), terminating the reactions after either 5, 10, 15, 20, 25, 30, 35, and 40 cycles in the Perkin Elmer 9600 PCR System Thermocycler.

### Hybridization

After amplification, 10 µL of the PCR admixture were combined with 10 microliters of a suspension containing about 10⁵ 3.5 micron diameter particles (Bangs Labs, Fishers, IN), previously coupled to the oligonucleotide probe (SEQ ID: NO: 6) suspended in 2x hybridization buffer (0.3M potassium chloride, 0.02M Tris and 2mM EDTA, pH 8.3), and the mixture was then heated for 3 minutes at 96°C, then allowed to cool to 65°C and incubated for ten minutes.

### Microparticle-immobilized DNA probe

The oligonucleotide probe immobilized on the particle has the sequence:
SEQ ID NO:6:
   5'-CTGCGTTAGA CCGAGAACTG TGGATAAAGG-3'

SEQ ID NO:6: was modified by covalent attachment of biotin to the 3' terminus. The probe was allowed to bind to streptavidin-coated particles, 3.5 micrometer in diameter (Bangs Laboratories) using standard protocols.

### DNA Staining

DNA staining was carried out by combining 500 µL of a 1:200 dilution of concentrated picogreen fluorescent dye with 20 µL of bead suspension comprising hybridized target and incubating the suspension for a minimum of 2 minutes at room temperature. Samples were next analyzed with the CYTORONABSOLUTE® flow cytometer.

### Results

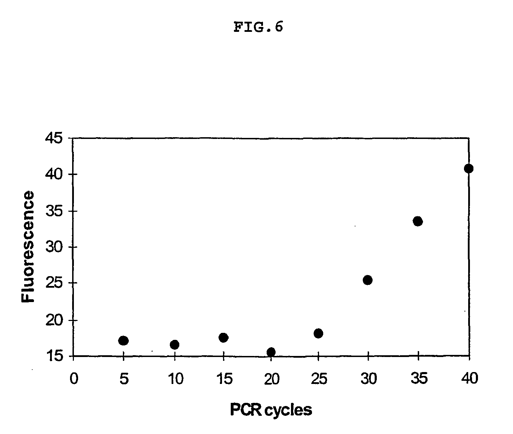

PCR product was measured as mean channel fluorescence derived from picogreen bound to hybridized target and particle-immobilized oligonucleotide dsDNA. In Fig. 6, it can be seen that the mean channel fluorescence increased with increasing PCR cycle number after 30 cycles demonstrating quantitative detection of PCR amplification product.

### Example 7

### Laser Scanning Cytometry

Laser scanning cytometry can be used in practicing the present invention. In a laser scanning cytometer, such as the Compucyte Laser Scanning Cytometer, an optical detector moves past substances dispersed, usually uniformly, on a surface in two spatial dimensions, for instance, on the surface of a microscope slide. This differs from flow microfluorimetry wherein particles move past a detector in one dimension (substantially one at a time in a moving stream).

Particles comprising a target-specific oligonucleotide probe must be large enough to be resolved by the detection system used in the laser scanning cytometer. Particles are preferably greater than or equal to about 0.3 microns, more preferably between about 1 to about 5 microns. The particles, sample comprising target nucleic acid and a compound that produces a detectable fluorescence when bound to dsDNA are combined to form a mixture. The mixture is diluted sufficiently to allow uniform dispersion of the particles on a microscope slide. The laser scanner moves over the slide and uses light scattering and/or fluorescence of the particles to trigger measurement of detectable fluorescence from a compound that is bound to hybridized target and particle-immobilized oligonucleotide. The laser scanning cytometer differentiates a plurality of distinct target-specific particles by the specific light scattering and/or fluorescence characteristics of the target-specific particles.

### Example 8

### DNA Arrays

In laser scanning cytometry and flow microfluorimetry, analyte detection relies on spatial resolution. In flow cytometry, resolution is one dimension. In laser scanning cytometry resolution is in two dimensions.

A DNA chip comprising oligonucleotide arrays represents a two-dimensional separation device. Methods for preparing such arrays are described in WO9818961 and in the U.S. Patents noted in the Background section.

In operation, a range of measurements, x1, is made in one direction, the x direction, and a range of measurements, y1, is made in a direction orthogonal to x, the y direction. Events detected as xly1 are known to be associated with a particular target-specific probe. Similarly, x2y2 measurements are known to be associated with a probe for the same target or for a different target, and so on.

DNA chips or slides comprising oligonucleotide arrays can be prepared by depositing and anchoring oligonucleotides directly to the surface or indirectly through chemical linkers or other immobilizing agents, all using techniques known in the art and found in WO 9818961, as well as the US Patents noted heretofore. They are immobilized in discrete xy locations on one surface of a chip or slide. Each xy locus, or spot, is specific for any desired target nucleic acid. Multiple spots specific to a single target can be located on the chip or slide to effect assay replication (replicate spots). Any desired number of target-specific spots and replicate spots can be used. Preferably, the chip or slide comprises between about 5 to about 20 target-specific spots and about the same number of replicate spots, more preferably the chip or slide comprises between about 100 to about 1000 target-specific spots and 10 to 100 replicate spots for each target. The DNA chip or slide is contacted with sample, and a compound that produces a detectable fluorescence signal when bound to dsDNA. The target-correlated fluorescence originating from the spots, that is, each specific xy location on the chip or slide is measured, for example, using a laser scanning device. The fluorescence signal is related to the presence or amount of the specific target nucleic acid.

In particular, a DNA chip having about 100 target-specific loci and about 10 replicate loci to SEQ. ID NO. 1, which oligonucleotide sequences are printed on silylated slides (CEL Associates). The print spots are about 125 µm in diameter and are spaced 300 µm apart from center to center. About 10 replicate probes to irrelevant sequence (for example, probes to plant genes where mammalian sequences are detected) are also printed on the slides. Printed glass slides are treated with sodium borohydrate solution (0.066M NaBH4, 0.06M Na AC) to ensure amino-linkage of probes to the slides. The slides are then boiled in water for 2 minutes to denature the cDNA. A biological containing from about 30 to about 30,000 target nucleic molecules is heated to 99°C for 5 minutes, then pre-cooled before hybridization, in this case, held at room temperature for 5 minutes, and then applied to the slides. The slides are covered with glass cover slips, sealed with DPX (Fluka) and hybridized at 60°C for 4-6 hours. At the end of hybridization slides are cooled to room temperature. The slides are washed in 1X SSC, 0.2% SDS at 55°C for 5 minutes, 0.1X SSC, 0.2% SDS at 55°C for 5 minutes. The slides are stained by contacting the entire surface with a 1:200 dilution of concentrated picogreen fluorescent dye and incubating for a minimum of 2 minutes at room temperature. After a quick rinse in 0.1X SSC, 0.2% SDS, the slides are air-blown dried and ready for scanning. Arrays are scanned for picogreen dye fluorescence using the ScanArray 3000 (General Scanning, Inc.). ImaGene Software (Biodiscovery, Inc.) is subsequently used for quantitation. The intensity of each spot is corrected by subtracting the immediate surrounding background.

The fluorescent signal is related to the presence or amount of the specific target nucleic acid and the slide has performed 10 replicates of this assay with mean of fluorescence from replicate test spots compared using statistical methods to mean of fluorescence from replicate probes to irrelevant sequence.

The above methods and procedures are repeated using a DNA chip having about 100 target-specific loci and about 10 replicate loci to SEQ. ID NO. 1, SEQ. ID NO. 2, SEQ. ID NO. 3, SEQ. ID NO 4, SEQ. ID NO. 5 and SEQ. ID NO.6.

### Example 9

### Detection of PCR Amplification Products: Homogeneous method

The purpose of this Example is to perform PCR thermocycling with IPC-IP beads in the PCR mix. Addition of DNA stain follows thermocycling.

The materials and procedures of Example 6 are employed, using the conditions, primers and probes identified below. Target DNA (10 copies), SEQ. ID No. 3, is amplified in a volume of 100 microliters using suitable PCR protocols that includes components of a PCR admixture, 0.25 microliter of 10⁵ oligonucleotide-bound 1.7 micron beads. Beads used are poly[styrene-co(p-vinylbenzylthio)proprionic acid] 95:5 molar ratio beads prepared as described in U.S. Patent No. 5,149,737; 5,210,289 and 5,278,267, and the appropriate concentration of a DNA-binding fluorophore, in this case, 500 microliter of a 1:200 dilution of concentrated picogreen, which is added following the thermocycling. Amplification reactions are set up in PCR conditions of Example 6, except 10⁵ oligonucleotide-bound microparticles is included in the admixture and a temperature cycle allowing for hybridization after the PCR cycles is added.

The presence of the amplified target DNA in the resulting mixture is then determined by flow cytometry using, for example, the CYTORONABSOLUTE® flow cytometer.

### Target DNA

A target DNA having the following sequence is cloned in pUC18:

SEQ ID NO.3:

5'-CTGCAGGCGC CAGCGTGGAC CATCAAGTAG TAATGAACGC
ACGGACGAGG ACATCATAGA GATTACACCT TTATCCACAG TTCTCGGTCT
AACGCAGCAG TCAGTGTATC AGCACCAGCA TCCGTAGTGA GTCTTCAGTG
TCTGCTCCAG GATCGTGGCG CTGCAG-3'

### Amplification and Hybridization reactions

Using PCR conditions and primers identified below, the sequences underlined in SEQ ID NO.3: are amplified using modified amplification protocols (cycles 1-5: 30s at 96°C: 60s at 68°C.; cycles 6-40: 15s at 96°C; 60s at 68°C; cycle 41: 5 minutes at 72°C; cycle 42: 3 minutes at 96°C; cycle 43: 60s at 50°C) in the PE9600 Thermocycler.
SEQ ID NO.4: (forward primer)
5'-CGCCAGCGT GGACCATCA AGTAGTAA-3'
SEQ ID NO.5: (reverse primer)
5-'CACGATCCT GGAGCAGAC ACTGAAGA-3'

The oligonucleotide probe immobilized on the particle has the sequence:

SEQ ID NO.6:
5'-CTGCGTTAG ACCGAGAAC TGTGGATAA AGG-3'

SEQ ID NO.6: is modified by covalent attachment to the surface of polystyrene particles, 1 micrometer in diameter using standard protocols.

### DNA Staining

DNA staining is carried out by combining 500 µL of a 1:200 dilution of concentrated picogreen with 20 µL of the above-identified PCR bead suspension comprising hybridized amplified target, and incubating the suspension for a minimum of 2 minutes at room temperature

### Results

PCR product is measured as mean channel fluorescence derived from picogreen bound to hybridized target and particle-immobilized oligonucleotide dsDNA.

### EXAMPLE 10

### Detection of PCR Amplification Products: Homogeneous method

The purpose of this Example is to perform PCR thermocycling with beads and DNA dye in the PCR mix.

The materials and procedures of Example 9 are employed except that 2.5 microliters of concentrated thermostable dye (for example, SYBR Green I dye, Molecular Probes, Eugene, Oregon) is added to the PCR suspension containing target, prior to thermocycling. Other thermostable dyes that can be used include ethidium bromide and propidium iodide.

### Results

PCR product is measured as mean channel fluorescence derived from SYBR Green bound to hybridized target and particle-immobilized oligonucleotide dsDNA.

### EXAMPLE 11

### Detection of PCR Amplification Products: Homogeneous method

The purpose of this Example is to perform PCR thermocycling in the presence of thermostable DNA dye in the PCR mix. Addition of microbeads follows thermocycling.

The materials and procedures of Example 9 are employed with 2.5 microliters of a concentrated thermostable SYBR Green dye added to the PCR target suspension prior to thermocycling. Other thermostable dyes that can be used include ethidium bromide and propidium iodide.

### DNA Hybridization

Following thermocycling, 10 microliters of 10⁵ oligonucleotide-bound microparticles (1 to 10 microns in size) suspended in 2x hybridization buffer (0.3M potassium chloride, 0.02M Tris and 2mM EDTA, pH 8.3), are added to 10 microliters of the amplified product-dye admixture, and heated for 3 minutes at 96°C, then allowed to cool to 65°C to allow hybridization.

### Analysis

The hybridized samples are supplemented with 500 µL of 1mM Tris, 10mM EDTA buffer and analyzed with for example, the CYTORONABSOLUTE® flow cytometer.

### Results

PCR product is measured as mean channel fluorescence derived from SYBR Green bound to hybridized target and particle-immobilized oligonucleotide dsDNA.

## Claims

1. A method for determining the presence or amount of a target nucleic acid comprising:
(A) forming a mixture comprising
i) the target nucleic acid,
ii) target-specific particles, wherein a target-specific particle has immobilized thereto an oligonucleotide comprising a nucleic acid sequence complementary to at least a portion of the target nucleic acid, wherein the oligonucleotide comprises a second compound capable of fluorescence and the particle or the oligonucleotide comprises a fluorescence quenching compound in sufficient proximity to said second compound to quench the fluorescence of said second compound prior to hybridization of the oligonucleotide to the target nucleic acid, and wherein the particle
(a) comprises a first compound or plurality of compounds capable of producing a distinct fluorescence signal corresponding to the particle, or
(b) is capable of scattering electromagnetic radiation of wavelength greater than or equal to about 200nm and comprises a first compound or plurality of compounds capable of producing a distinct fluorescence signal corresponding to the particle;
(B) heating the mixture to denature duplex nucleic acid then cooling the mixture to allow hybridization of target nucleic acid to immobilized oligonucleotide;
(C) exposing the mixture to a detection device capable of detecting fluorescence and, optionally, scattered electromagnetic radiation;
(D) detecting the fluorescence of a first compound or a plurality of compounds of a particle, or detecting both the fluorescence of a first compound or a plurality of compounds of a particle and the scattered electromagnetic radiation of a particle; and
(E) detecting the fluorescence signal of the second compound as a measure of the presence or amount of the target nucleic acid.

2. A method for determining the presence or amount of a plurality of distinct target nucleic acids comprising:
(A) forming a mixture comprising
i) the target nucleic acids,
ii) target-specific particles, wherein a target-specific particle has immobilized thereto an oligonucleotide comprising a nucleic acid sequence complementary to at least a portion of one distinct target nucleic acid, wherein the oligonucleotide comprises a second compound capable of fluorescence and the particle or the oligonucleotide comprises a fluorescence quenching compound in sufficient proximity to said second compound to quench the fluorescence of said second compound prior to hybridization of the oligonucleotide to the target nucleic acid, and wherein the particle
(a) comprises a distinct first compound or plurality of compounds capable of producing a distinct fluorescence signal corresponding to the particle, or
(b) is capable of scattering electromagnetic radiation of wavelength greater than or equal to about 200nm and comprises a distinct first compound or plurality of compounds capable of producing a distinct fluorescence signal corresponding to the distinct particle,
iii) a compound capable of binding to duplex nucleic acid and producing a detectable optical signal when bound thereto;
(B) heating the mixture to denature duplex nucleic acid then cooling the mixture to allow hybridization of target nucleic acid to immobilized oligonucleotide;
(C) exposing the mixture to a detection device capable of detecting fluorescence and, optionally, scattered electromagnetic radiation;
(D) detecting the fluorescence of a first compound or a plurality of compounds of a particle, or detecting both the fluorescence of a first compound or a plurality of compounds of a particle and the scattered electromagnetic radiation of a particle; and
(E) detecting the fluorescence signal of the second compound as a measure of the presence or amount of each distinct target nucleic acid.

3. A kit for detecting a target nucleic acid comprising in the same or separate containers:
(A) a particle
(a) comprising a compound or plurality of compounds capable of producing a fluorescence signal corresponding to the particle, or
(b) capable of scattering electromagnetic radiation of wavelength greater than or equal to about 200nm and comprising a compound or plurality of compounds capable of producing a fluorescence signal corresponding to the particle; and
(B) an oligonucleotide comprising
(a) a nucleic acid sequence complementary to at least a portion of the target nucleic acid,
(b) a first compound capable of fluorescence, and
(c) a second compound capable of quenching the fluorescence of the first compound.

4. The method of claim 2, wherein the compound capable of binding to duplex nucleic acid and producing a detectable optical signal when bound thereto is selected from the group consisting of SYBR green I, ethidium bromide, propidium iodide, acridine orange, mithramycin, chromomycin, olivomycin, Hoechst H33258, Hoechst H33342, DAPI (4', 6diamidino-2-phenylindole), TOPRO, TOTO, YOPRO, YOYO, and Picogreen.

5. The method of claims 1 or 2, wherein the detection device is a flow cytometer, a scanning cytometer, or a fluorescence microscope.

## Patentansprüche

1. Verfahren zur Bestimmung des Vorhandenseins oder der Menge einer Zielnukleinsäure, umfassend:
(A) Bilden einer Mischung, umfassend
i) die Zielnukleinsäure,
ii) zielspezifische Teilchen, wobei ein zielspezifisches Teilchen daran immobilisiert ein Oligonukleotid aufweist, das eine Nukleinsäuresequenz umfasst, die zu wenigstens einem Abschnitt der Zielnukleinsäure komplementär ist, wobei das Oligonukleotid eine zweite Verbindung umfasst, die zur Fluoreszenz in der Lage ist, und das Teilchen oder das Oligonukleotid eine fluoreszenzlöschende Verbindung in ausreichender Nähe zur zweiten Verbindung umfasst, um die Fluoreszenz der zweiten Verbindung vor der Hybridisierung des Oligonukleotids an die Zielnukleinsäure zu löschen, und wobei das Teilchen
(a) eine erste Verbindung oder Mehrzahl von Verbindungen umfasst, die ein verschiedenes Fluoreszenzsignal erzeugen kann/können, das dem Teilchen entspricht, oder
(b) elektromagnetische Strahlung einer Wellenlänge streuen kann, die größer als oder gleich etwa 200 nm ist, und eine erste Verbindung oder Mehrzahl von Verbindungen umfasst, die ein verschiedenes Fluoreszenzsignal erzeugen kann/können, das dem Teilchen entspricht;
(B) Erhitzen der Mischung, um Duplex-Nukleinsäure zu denaturieren, und anschließendes Abkühlen der Mischung, um Hybridisierung von Zielnukleinsäure an immobilisiertes Oligonukleotid zu ermöglichen;
(C) Exponieren der Mischung gegenüber einer Nachweisvorrichtung, die Fluoreszenz und gegebenenfalls gestreute elektromagnetische Strahlung nachweisen kann;
(D) Nachweisen der Fluoreszenz einer ersten Verbindung oder einer Mehrzahl von Verbindungen eines Teilchens oder Nachweisen sowohl der Fluoreszenz einer ersten Verbindung oder einer Mehrzahl von Verbindungen eines Teilchens als auch der gestreuten elektromagnetischen Strahlung eines Teilchens; und
(E) Nachweisen des Fluoreszenzsignals der zweiten Verbindung als ein Maß des Vorhandenseins oder der Menge der Zielnukleinsäure.

2. Verfahren zur Bestimmung des Vorhandenseins oder der Menge mehrerer verschiedener Zielnukleinsäuren, umfassend:
(A) Bilden einer Mischung, umfassend
i) die Zielnukleinsäuren,
ii) zielspezifische Teilchen, wobei ein zielspezifisches Teilchen daran immobilisiert ein Oligonukleotid aufweist, das eine Nukleinsäuresequenz umfasst, die zu wenigstens einem Abschnitt einer verschiedenen Zielnukleinsäure komplementär ist, wobei das Oligonukleotid eine zweite Verbindung umfasst, die zur Fluoreszenz in der Lage ist, und das Teilchen oder das Oligonukleotid eine fluoreszenzlöschende Verbindung in ausreichender Nähe zur zweiten Verbindung umfasst, um die Fluoreszenz der zweiten Verbindung vor der Hybridisierung des Oligonukleotids an die Zielnukleinsäure zu löschen, und wobei das Teilchen
(a) eine verschiedene erste Verbindung oder Mehrzahl von Verbindungen umfasst, die ein verschiedenes Fluoreszenzsignal erzeugen kann/können, das dem Teilchen entspricht, oder
(b) elektromagnetische Strahlung einer Wellenlänge streuen kann, die größer als oder gleich etwa 200 nm ist, und eine verschiedene erste Verbindung oder Mehrzahl von Verbindungen umfasst, die ein verschiedenes Fluoreszenzsignal erzeugen kann/können, das dem verschiedenen Teilchen entspricht;
iii) eine Verbindung, die an Duplex-Nukleinsäure binden und ein nachweisbares optisches Signal erzeugen kann, wenn sie daran gebunden ist;
(B) Erhitzen der Mischung, um Duplex-Nukleinsäure zu denaturieren, anschließendes Abkühlen der Mischung, um Hybridiserung von Zielnukleinsäure an immobilisiertes Oligonukleotid zu ermöglichen;
(C) Exponieren der Mischung gegenüber einer Nachweisvorrichtung, die Fluoreszenz und gegebenenfalls gestreute elektromagnetische Strahlung nachweisen kann;
(D) Nachweisen der Fluoreszenz einer ersten Verbindung oder einer Mehrzahl von Verbindungen eines Teilchens oder Nachweisen von sowohl der Fluoreszenz einer ersten Verbindung oder einer Mehrzahl von Verbindungen eines Teilchens als auch der gestreuten elektromagnetischen Strahlung eines Teilchens; und
(E) Nachweisen des Fluoreszenzsignals der zweiten Verbindung als ein Maß des Vorhandenseins oder der Menge jeder verschiedenen Zielnukleinsäure.

3. Kit zum Nachweis einer Zielnukleinsäure, der im selben oder getrennten Behältern umfasst:
(A) ein Teilchen,
(a) das eine Verbindung oder Mehrzahl von Verbindungen umfasst, die ein Fluoreszenzsignal erzeugen kann/können, das dem Teilchen entspricht, oder
(b) elektromagnetische Strahlung einer Wellenlänge streuen kann, die größer als oder gleich etwa 200 nm ist, und eine Verbindung oder Mehrzahl von Verbindungen umfasst, die ein Fluoreszenzsignal erzeugen kann/können, das dem Teilchen entspricht; und
(B) ein Oligonukleotid, umfassend
(a) eine Nukleinsäuresequenz, die zu wenigstens einem Abschnitt der Zielnukleinsäure komplementär ist,
(b) eine erste Verbindung, die zur Fluoreszenz in der Lage ist, und
(c) eine zweite Verbindung, die die Fluoreszenz der ersten Verbindung löschen kann.

4. Verfahren nach Anspruch 2, wobei die Verbindung, die an Duplex-Nukleinsäure binden und ein nachweisbares optisches Signal erzeugen kann, wenn sie daran gebunden ist, ausgewählt ist aus der Gruppe, bestehend aus SYBR-Grün I, Ethidiumbromid, Propidiumiodid, Acridinorange, Mithramycin, Chromomycin, Olivomycin, Hoechst H33258, Hoechst H33342, DAPI (4',6-Diamidino-2-phenylindol), TORPO, TOTO, YOPRO, YOYO und Picogreen.

5. Verfahren nach den Ansprüchen 1 oder 2, wobei die Nachweisvorrichtung ein Durchflusscytometer, ein Scanningcytometer oder ein Fluoreszenzmikroskop ist.

## Revendications

1. Procédé pour déterminer la présence ou la quantité d'un acide nucléique cible comprenant les étapes consistant à :
(A) former un mélange comprenant
(i) l'acide nucléique cible,
(ii) des particules spécifiques de la cible, où une particule spécifique de l'acide a un oligonucléotide immobilisé comprenant une séquence d'acides nucléiques complémentaire d'au moins une partie de l'acide nucléique cible, où l'oligonucléotide comprend un second composé capable de fluorescence et la particule ou l'oligonucléotide comprend un composé d'extinction de fluorescence à proximité suffisante dudit second composé pour éteindre la fluorescence dudit second composé avant hybridation de l'oligonucléotide sur l'acide nucléique cible et où la particule
(a) comprend un premier composé ou une pluralité de composés capables de produire un signal de fluorescence distinct correspondant à la particule, ou
(b) est capable de diffuser un rayonnement électromagnétique de longueur d'onde supérieure ou égale à environ 200 nm et comprend un premier composé ou une pluralité de composés capables de produire un signal de fluorescence distinct correspondant à la particule ;
(B) chauffer le mélange pour dénaturer un acide nucléique duplexe puis refroidir le mélange pour permettre l'hybridation de l'acide nucléique cible sur l'oligonucléotide immobilisé ;
(C) exposer le mélange à un dispositif de détection capable de détecter une fluorescence et, facultativement, un rayonnement électromagnétique diffusé ;
(D) détecter la fluorescence d'un premier composé et d'une pluralité de composés d'une particule, ou détecter à la fois la fluorescence d'un premier composé ou d'une pluralité de composés d'une particule et le rayonnement électromagnétique diffusé d'une particule ; et
(E) détecter le signal de fluorescence du second composé comme une mesure de la présence de la quantité de l'acide nucléique cible.

2. Procédé pour déterminer la présence ou la quantité d'une pluralité d'acides nucléiques cibles distinctes comprenant les étapes consistant à :
(A) former un mélange comprenant
(i) les acides nucléiques cibles,
(ii) des particules spécifiques de la cible, où une particule spécifique de l'acide a un oligonucléotide immobilisé comprenant une séquence d'acides nucléiques complémentaire d'au moins une partie de l'acide nucléique cible, où l'oligonucléotide comprend un second composé capable de fluorescence et la particule ou l'oligonucléotide comprend un composé d'extinction de fluorescence à proximité suffisante dudit second composé pour éteindre la fluorescence dudit second composé avant hybridation de l'oligonucléotide sur l'acide nucléique cible et où la particule
(a) comprend un premier composé ou une pluralité de composés capables de produire un signal de fluorescence distinct correspondant à la particule, ou
(b) est capable de diffuser un rayonnement électromagnétique de longueur d'onde supérieure ou égale à environ 200 nm et comprend un premier composé ou une pluralité de composés capables de produire un signal de fluorescence distinct correspondant à la particule distincte,
(iii) un composé capable de se lier à un acide nucléique duplexe et à produire un signal optique lorsqu'il lui est lié ;
(B) chauffer le mélange pour dénaturer un acide nucléique duplexe puis refroidir le mélange pour permettre l'hybridation de l'acide nucléique cible sur l'oligonucléotide immobilisé ;
(C) exposer le mélange à un dispositif de détection capable de détecter une fluorescence et, facultativement, un rayonnement électromagnétique diffusé ;
(D) détecter la fluorescence d'un premier composé et d'une pluralité de composés d'une particule, ou détecter à la fois la fluorescence d'un premier composé ou d'une pluralité de composés d'une particule et le rayonnement électromagnétique diffusé d'une particule ; et
(E) détecter le signal de fluorescence du second composé comme une mesure de la présence de la quantité de chaque acide nucléique cible distinct.

3. Nécessaire pour détecter un acide nucléique cible comprenant dans des contenants identiques ou séparées :
(A) une particule
(a) comprenant un composé d'une pluralité de composés capables de produire un signal de fluorescence correspondant à la particule, ou
(b) capable de diffuser un rayonnement électromagnétique de longueur d'onde supérieure ou égale à 200 nm et comprenant un composé ou une pluralité de composés capables de produire un signal de fluorescence correspondant à la particule ; et
(B) un oligonucléotide comprenant
(a) une séquence d'acides nucléiques complémentaire d'au moins une partie de l'acide nucléique cible,
(b) un premier composé capable de fluorescence, et
(c) un second composé capable d'éteindre la fluorescence du premier composé.

4. Procédé selon la revendication 2, dans lequel le composé capable de se lier à un acide nucléique duplexe et de produire un signal optique détectable lorsqu'il lui est lié est choisi dans le groupe consistant en vert I SYBR, bromure d'éthidium, iodure de propidium, orange acridine, mithramycine, chromomycine, olivomycine, Hoechst H33258, Hoechst H33342, DAPI (4', 6-diamidino-2-phénylindole), TOPRO, TOTO, YOPRO, YOYO et picovert.

5. Procédé selon les revendications 1 ou 2, dans lequel le dispositif de détection est un cytomètre en flux, un cytomètre à balayage ou un microscope à fluorescence.
